(19) **Europäisches Patentamt**

**European Patent Office**

**Office européen des brevets**

(11) **EP 1 007 961 B1**

(12) **FASCICULE DE BREVET EUROPEEN**

(45) Date de publication et mention
de la délivrance du brevet:
**12.11.2003 Bulletin 2003/46**

(51) Int Cl.$^7$: **G01N 33/00**

(86) Numéro de dépôt international:
**PCT/FR98/01896**

(21) Numéro de dépôt: **98942826.3**

(22) Date de dépôt: **04.09.1998**

(87) Numéro de publication internationale:
**WO 99/012028 (11.03.1999 Gazette 1999/10)**

(54) **APPAREIL DE CLASSIFICATION DESTINE NOTAMMENT A LA RECONNAISSANCE D'ODEURS**

KLASSIFIZIERANLAGE FÜR GERUCHSERKENNUNG

CLASSIFYING APPARATUS DESIGNED IN PARTICULAR FOR ODOUR RECOGNITION

(84) Etats contractants désignés:
**DE FR GB**

(30) Priorité: **04.09.1997 FR 9711000**

(43) Date de publication de la demande:
**14.06.2000 Bulletin 2000/24**

(73) Titulaire: **ALPHA M.O.S.**
**31400 Toulouse (FR)**

(72) Inventeurs:
• **LABRECHE, Said**
**F-31200 Toulouse (FR)**
• **AMINE, Hicham**
**F-31400 Balma (FR)**
• **TAN, Tze, Tsung**
**F-31500 Toulouse (FR)**
• **LOUBET, François**
**F-31130 Balma (FR)**

(74) Mandataire: **Joly, Jean-Jacques et al**
**Cabinet Beau de Loménie**
**158, rue de l'Université**
**75340 Paris Cédex 07 (FR)**

(56) Documents cités:
**EP-A- 0 632 268**

• **RATTON L ET AL: "A comparative study of signal processing techniques for clustering microsensor data (a first step towards an artificial nose)" SENSORS AND ACTUATORS B, vol. 41, no. 1-3, 30 juin 1997, page 105-120 XP004089128**
• **HORNER G: "SIGNALVERARBEITUNG BEI CHEMOSENSORARRAYS" TECHNISCHES MESSEN TM, vol. 62, no. 4, 1 avril 1995, pages 166-172, XP000516380**
• **WANG PING ET AL: "A NOVEL METHOD COMBINED NEURAL NETWORK WITH FUZZY LOGIC FOR ODOUR RECOGNITION" MEASUREMENT SCIENCE AND TECHNOLOGY, vol. 7, no. 12, décembre 1996, pages 1707-1712, XP000681919**
• **CRAVEN M A ET AL: "ELECTRONIC NOSES-DEVELOPMENT AND FUTURE PROSPECTS" TRAC, TRENDS IN ANALYTICAL CHEMISTRY, vol. 15, no. 9, octobre 1996, pages 486-493, XP000641956**

**Description**

La présente invention concerne la classification de motifs, notamment dans le domaine de la caractérisation ou de l'identification d'odeurs et, plus particulièrement, un appareil de classification de motifs qui sélectionne dans un répertoire la règle de classification appropriée en fonction de l'application.

Dans les appareils de caractérisation ou d'identification d'odeurs (dits "nez électroniques") un échantillon d'un produit inconnu est identifié en le classant, selon une règle de classification, dans une classe parmi plusieurs classes que l'appareil reconnaît grâce à une opération d'apprentissage préalable pendant laquelle les caractéristiques des classes ont été déterminées.

Les appareils de ce genre comportent deux parties principales, une partie d'acquisition de données et une partie de traitement des données. La partie d'acquisition de données comporte, en général, une tête de prélèvement permettant de prélever des composants volatiles qui se dégagent du produit testé, et une batterie de capteurs auxquels sont fournis les composants prélevés et qui produisent des signaux de sortie qui, pris en combinaison, sont caractéristiques des composants volatiles dégagés par le produit et donc sont caractéristiques du produit. La partie de traitement de données comporte, en général, des moyens informatiques qui enregistrent et effectuent des analyses sur les signaux de sortie des capteurs. En décelant ou analysant les motifs inhérents aux différents ensembles de données relatifs à des produits particuliers, la partie de traitement de données détecte la classe du produit, qui peut correspondre soit à la nature du produit (par exemple, "un vin"; "un whisky"; etc.), soit à une concentration ou une qualité du produit, par exemple, "lait frais"; "lait fermenté"; etc..

Les objectifs poursuivis en acquérant et en traitant les données sont différents selon que l'appareil est en mode d'apprentissage ou en mode d'identification.

Dans la phase d'apprentissage de l'appareil, les produits dont sont extraits les différents échantillons sont connus et analysés plusieurs fois. Les méthodes de traitement utilisées sont alors celles qui permettent d'une part de détecter et caractériser les différents produits et d'autre part de construire un modèle robuste d'identification. En général un modèle d'identification consiste en deux éléments, une méthode d'extraction d'information à partir des signaux de sortie des capteurs et une règle d'identification permettant de classer dans une classe particulière des données obtenues pour un échantillon de nature inconnue. Les méthodes d'extraction d'information utilisées sont généralement des méthodes statistiques, notamment une Analyse en Composantes Principales (ACP) ou Analyse Factorielle Discriminante (AFD) (voir par exemple, J.W. Gardner et P.N. Bartlett," Sensors and sensory systems form an Electronic nose", Nato Asi, Series E Vol. 212, pp. 161-180 (1992)). Dans la phase d'identification, la nature des échantillons n'est pas connue et l'objectif est d'identifier cette nature en utilisant les données et le modèle déjà construit.

Typiquement, les appareils de ce genre mettent en oeuvre une analyse de type fixe des données obtenues au cours de la phase d'apprentissage afin d'arriver à certaines définitions des classes et, au cours de la phase d'identification, ils appliquent une règle fixe pour affecter des échantillons de produits inconnus à l'une, appropriée, des classes définies.

La présente invention à pour objet un appareil de classification de motifs, destiné notamment à une utilisation dans le domaine de la reconnaissance d'odeurs, dans lequel il y a un choix entre plusieurs régies d'affectation différentes, et, ou bien, entre plusieurs méthodes d'extraction d'informations différentes, afin de permettre au modèle d'identification d'être adapté à l'application visée.

Plus particulièrement, la présente invention prévoit un appareil de classification, destiné notamment à une utilisation dans la reconnaissance ou la caractérisation d'odeurs, comprenant : un moyen destiné à acquérir, par une pluralité de canaux, au cours d'une phase d'apprentissage, des données brutes représentant une pluralité d'instances d'une pluralité de classes différentes, l'affectation aux classes respectives des instances analysées au cours de la phase d'apprentissage étant connue ; une unité de traitement destinée à traiter les données fournies par le moyen d'acquisition de données pour déterminer, au cours de la phase d'apprentissage, un modèle d'identification définissant les classes différentes et, pendant une phase d'identification, à classer selon une règle particulière une instance de classe inconnue dans une des classes définies au cours de la phase d'apprentissage : caractérisé en ce que l'unité de traitement est adaptée à décider lequel d'une pluralité de modèles d'identification possibles, dont les caractéristiques sont stockés dans un répertoire, doit être appliqué.

En choisissant la règle d'affectation et, ou bien, la méthode d'extraction d'informations de façon dynamique, en fonction de l'application visée, l'appareil de la présente invention atteint un taux de reconnaissance de produits inconnus amélioré par rapport aux appareils classiques.

Il est préférable de sélectionner de façon dynamique à la fois la règle d'affectation et la méthode utilisée pour extraire des informations des données brutes mesurées. Tous deux de ces éléments font partie du modèle d'identification établi au cours de la phase d'apprentissage de l'appareil. De préférence, l'unité de traitement des données brutes utilise en tant que méthode d'extraction d'informations des traitements statistiques tels qu'une analyse en composantes principales ou une analyse factorielle discriminante. En général, ces méthodes servent à déterminer des sous-espaces respectifs dans lesquels les différentes classes sont bien discriminées les unes par rapport aux autres.

De préférence, plusieurs modèles d'identification différents sont déterminés sur la base d'un pourcentage seulement des données obtenues au cours de la phase d'apprentissage. Le modèle d'identification qui sera choisi est celui qui donne le meilleur taux de reconnaissance lorsque les autres données obtenues au cours de la phase d'apprentissage lui sont soumises.

Parmi les différentes règles d'affectation possibles se trouve une règle qui définit le centre de gravité des points représentant les instances d'une même classe et qui affecte un échantillon inconnu à la classe pour laquelle la distance entre le point représentant l'instance inconnue et le centre de gravité est un minimum. Il y a aussi une règle d'affectation selon laquelle on détermine la frontière délimitant les points représentant les instances d'une classe des points représentant les instances des autres classes, et une instance inconnue est affectée à la classe pour laquelle la dilatation de la frontière nécessaire pour englober le point correspondant à cette instance inconnue est un minimum. Une troisième règle d'affectation préférée consiste en l'établissement d'une distribution de probabilités, basée sur une somme pondérée des gausiennes représentant les instances des classes différentes, et une instance d'une classe inconnue est affectée à l'une des classes selon le positionnement du point la représentant par rapport à cette distribution de probabilités.

Dans certaines applications il peut s'avérer utile, ou bien nécessaire, d'éliminer des analyses les données brutes relatives aux canaux qui ne contribuent pas à différencier les instances de classes différentes. Il peut aussi s'avérer nécessaire d'éliminer des calculs les données concernant des instances aberrantes d'une classe, c'est-à-dire, des instances qui, en termes de variables synthétiques, sont éloignées des autres instances de la même classe. Ceci augmente la fiabilité du modèle d'identification établi pendant la phase d'apprentissage. De la même façon, des données obtenues au cours de la phase d'apprentissage vis-à-vis d'instances aberrantes d'une classe peuvent être supprimées.

On va maintenant décrire des modes de réalisation particuliers de la présente invention, à titre d'exemples non limitatifs, en liaison avec les dessins annexés dans lesquels:

la figure 1 est un organigramme illustrant les différentes étapes du traitement, servant à déterminer les caractéristiques des classes différentes, qui est appliqué dans le mode de réalisation préféré de l'invention ;

la figure 2 est un graphique montrant la distribution de données obtenues par une analyse en composantes principales et représentant des instances de cinq classes différentes dans un plan qui permet de différencier les différentes classes les unes des autres ;

la figure 3 est un graphique montrant des frontières définies, selon une règle d'identification de classes particulière, pour délimiter des régions correspondant aux classes différentes illustrées à la figure 2 ;

la figure 4 est un graphique montrant comment les frontières indiquées à la figure 3 peuvent être dilatées selon une variante de la règle d'identification concernée ;

la figure 5 est un graphique montrant une distribution de probabilités, utilisée par une autre règle d'identification selon l'invention, correspondant aux cinq classes montrées à la figure 2 ; et

la figure 6 est un organigramme illustrant les différentes étapes du procédé de sélection d'un modèle d'identification selon le mode de réalisation préféré de l'invention.

Des modes de réalisation préférés de l'appareil selon la présente invention seront maintenant décrits dans le contexte d'appareils de reconnaissance d'odeurs. Il est à comprendre, cependant, que la présente invention n'est pas limitée à de telles applications mais s'applique aussi bien à d'autre domaines où des données représentant des instances de classes différentes doivent être classées.

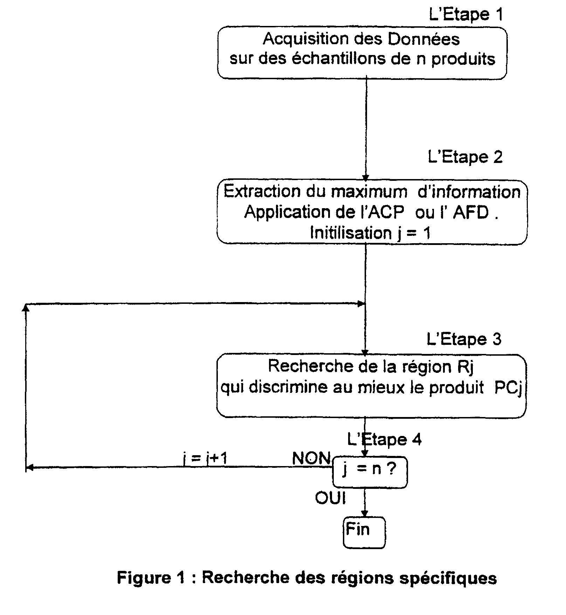

Un mode de réalisation préféré de la présente invention sera maintenant décrit à l'aide des figures 1 à 6. Ce mode de réalisation utilise un appareil du type dit "nez électronique" comportant $p$ capteurs. Ces capteurs peuvent inclure des capteurs de types classiques tels, par exemple. des capteurs à polymère conducteur, des capteurs piézo-électriques à quartz; des capteurs à oxyde(s) semi-conducteur(s), etc. Au cours de la phase d'apprentissage de l'appareil, plusieurs échantillons de produits connus sont présentés à l'appareil afin de générer des données brutes ( une base d'apprentissage) qui seront ensuite analysées pour établir un modèle d'identification. Ce modèle d'identification est ensuite utilisé pour classer des échantillons de produits inconnus par rapport aux classes établies au cours de la phase d'apprentissage.

Selon le mode de réalisation préféré de l'invention, plusieurs modèles d'identification sont disponibles. Au cours de la phase d'apprentissage, l'établissement du modèle d'identification passe par la sélection d'une méthode d'extraction d'informations permettant de déterminer les caractéristiques des différentes classes et par la sélection d'une règle d'affectation qui servira, au cours de la phase d'identification, à affecter des échantillons inconnus à l'une des classes établies. Ces choix s'effectuent en fonction de l'application visée, c'est-à-dire en fonction des caractéristiques des produits à identifier.

Avant de décrire le procédé de sélection de la méthode d'extraction et de la règle d'affectation selon l'invention, les méthodes d'extraction d'informations et les règles d'affectation préférées seront exposées.

## L'EXTRACTION DES INFORMATIONS

Plusieurs méthodes sont possibles pour extraire des informations à partir des données mesurées. Comme il est indiqué à la figure 1, au cours de la phase d'apprentissage, des données sont acquises (Etape 1) sur des échantillons de plusieurs produits connus. Ensuite, ces données sont traitées (Etape 2) afin d'en extraire des informations et de déterminer des caractéristiques communes aux différentes instances d'une même classe. Les méthodes d'extraction d'informations préférées sont l'Analyse en Composantes Principales et l'Analyse Factorielle Discriminante, qui permettent de représenter les différentes instances (échantillons) en tant que points respectifs dans un sous-espace de dimension réduite. En général, les points représentant les instances d'une même classe forment une nuage de points dans le sous-espace concerné. On peut, alors, définir dans le sous-espace (Etape 3) une région respective correspondant à chaque classe de sorte que les régions sont distinctes les unes des autres. En d'autres termes, la définition de régions assure que les classes sont bien discriminées les unes par rapport aux autres. Finalement, il est à vérifier que des régions de discrimination ont été définies pour chaque classe de produit utilisé dans la phase d'apprentissage (Etape 4).

Les différentes étapes de ce procédé seront maintenant décrites plus en détail.

### L'établissement d'une base d'apprentissage

Plusieurs capteurs sélectionnés sont utilisés dans un "nez électronique". A chaque échantillon analysé i, on associe un point $x_i$ dans un espace de dimension $p$, $p$ étant le nombre de capteurs.

$$x_i = \sum_{k=1}^{p} x(i,k)\, e_k \qquad\qquad \text{avec } k = 1 \text{ à } p$$

$e_k$ est un vecteur de dimension $p$ dont les composantes sont toutes égales à 0 sauf la $k^{eme}$ qui vaut 1. L'ensemble de ces vecteurs forme une base de l'espace. Ici, $X(i,k)$ est la mesure donnée par le $k^{eme}$ capteur lors de l'analyse de $i$. L'ensemble de ces représentations forme un nuage de points.

Généralement, lors de l'apprentissage de cet appareil, plusieurs échantillons sont analysés. Selon les applications, ces échantillons peuvent être extraits de différents produits (**PCJ** / $j$ =1 à $n$) ou d'un même produit à différents états (bon, mauvais, marginal) ou à différentes concentrations. A la fin de la première étape de la phase d'apprentissage (la phase "acquisition de données"), on obtient donc un tableau ou matrice $X$ de données de dimensions $(m,p)$, $m$ étant le nombre total d'échantillons analysés (c'est-à-dire que $i$ prend des valeurs comprises entre 1 et $m$).

### Traitement des données

Le but recherché est alors de déterminer si l'appareil permet :

- de discriminer les différents produits.
- d'identifier les échantillons d'un même produit.

Sur l'espace contenant les points, on définit une métrique $M$. On définit aussi certains éléments statistiques sur le tableau $X$ centré, en particulier les matrices des covariances $V$, des variances interclasse $B$, intraclasse $W$ et les centres de gravités ($g_j$ / $j$ = {1;$n$}, $n$ étant le nombre de produits analysés) des classes définies par les échantillons des différents produits (voir, par exemple, G. Saporta "Probabilités, Analyse des données et Statistique", Editions TECHNIP (1990) pour le calcul de ces éléments).

### Choix de capteurs

Au cours des prétraitements statistiques, les capteurs qui discriminent au mieux les différents produits sont choisis automatiquement. Ce choix est basé sur la recherche pas à pas de la meilleure combinaison. M.C. Costanza et AA Afifi ont proposé dans (Journal of the American Statistical Association. Vol. 74, Number 368 ( 1979)) un algorithme approprié. Les capteurs non choisis ne seront pas pris en compte dans toutes les analyses.

### Détermination de caractéristiques communes aux instances d'une classe

Comme il a déjà été mentionné ci-dessus, à chaque échantillon est associé un point $x_i$ dans un espace de dimension $p$ et, en général, les points représentant des instances d'une mème classe sont regroupés et les différents groupes de points représentant les classes différentes sont séparés les uns des autres. Le positionnement de chaque groupe de points dans le sous espace correspond. donc, en quelque sorte, à une définition de la classe. Or, définir des classes dans un sous-espace de dimension $p$ nécessite un traitement complexe et l'emploi d'expressions mathématiques compliquées. Des méthodes existent qui permettent d'extraire des données d'origine les informations permettant de discriminer les classes les unes des autres dans des sous-espaces de dimensions réduites. Les méthodes préférées consistent en une Analyse en Composantes Principales ou une Analyse Factorielle Discriminante. Dans chacune de ces méthodes, on cherche une nouvelle base qui optimise un critère mathématique bien défini (voir, par exemple. G. Saporta cité supra). L'application de ces méthodes permet d'associer à chaque échantillon un vecteur possédant un nombre de composantes réduit.

L'ACP permet de remplacer les capteurs choisis par de nouvelles variables synthétiques ($Cj, j = \{1;p\}$) qui discriminent au mieux les différents produits. En ACP, on cherche une base particulière ($cj, j = \{l;p\}$) de l'espace. La variable synthétique $Cj$ est alors associée au vecteur $cj$.

Les vecteurs ($cj, j = \{1,p\}$) sont les vecteurs propres $M$ orthonormés de la matrice $VM$. Voir, par exemple, Gene H. Golub & Charles F. Van Loan "Matrix Computations", The Johns Hopkins University Press (1990) pour le calcul des éléments propres. Les variables synthétiques sont alors définies par

$$Cj(x) \; x'Mcj$$

avec $j = \{l:p\}$, $x'$ étant la transposé de $x$. Généralement $M$ est associée à la matrice identité.

L'AFD, pour sa part, permet de remplacer les capteurs choisis par de nouvelles variables synthétiques ($Uj, j = 1;p\}$) qui discriminent au mieux les différents produits. En AFD, on cherche une base particulière ($uj,j = \{l;p\}$) de l'espace. La variable synthétique $Uj$ est alors associée au vecteur $uj$.

Les vecteurs ($uj, j = \{1;p\}$) sont les vecteurs propres $W^{-1}$ orthonormés de la matrice $BW^{-1}$. Voir supra Gene H. Golub & Charles F. Van Loan "Matrix Computations" pour le calcul des éléments propres. Les variables synthétiques sont alors définies par

$$Uj(x) = x'W^{-1}uj$$

avec $j = \{1;p\}$, $x'$ étant la transposée de $x$, et $W^{-1}$ l'inverse de la matrice $W$.

L'exploration des différents sous espaces engendrés par les nouvelles variables synthétiques $V$ (qu'elles soient déterminées par l'ACP ou par l'AFD ou par une autre méthode) permet de trouver le sous-espace $SEj$ dans lequel une classe (ici, un produit) est la mieux discriminée par rapport aux autres. Cette exploration peut se faire manuellement en visualisant les différents plans ou d'une manière automatique en cherchant la meilleure combinaison de ces variables.

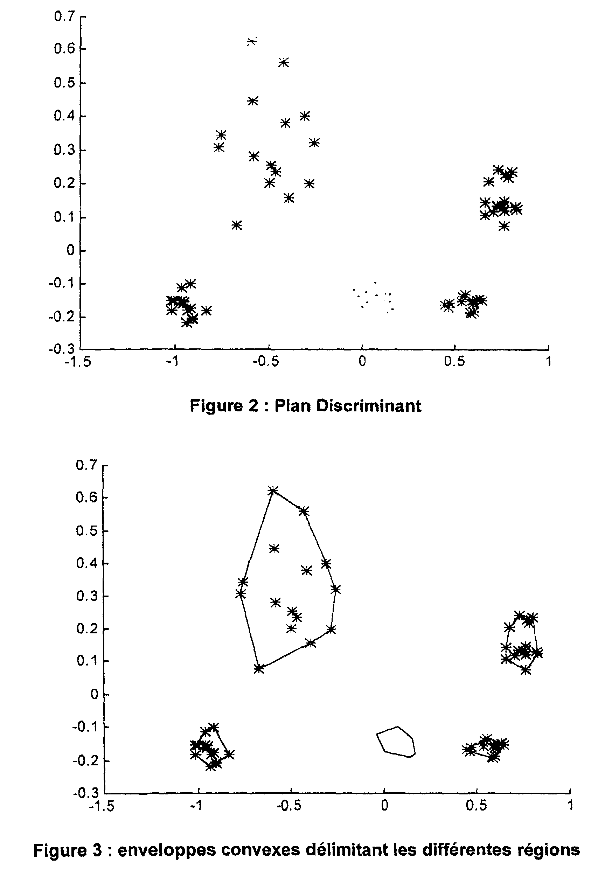

La figure 2 illustre un exemple des résultats d'une analyse typique et représente la projection des échantillons de cinq produits sur un plan défini par deux variables synthétiques obtenues par une ACP. On observe que les différents produits sont tous bien discriminés dans ce plan. Dans ce cas, tous les sous espaces $SEj$ sont confondus avec ce plan. Dans ce cas, on peut considérer que chaque classe correspond à une région $Rj$ de ce même sous-espace, les régions $Rj$ étant définies de manière à assurer le fait que chaque classe est discriminée des autres.

Par ailleurs, les méthodes de traitement statistiques évoquées ci-dessus permettent aussi de détecter les points aberrants dont la présence fausserait la définition de la classe correspondante. Un échantillon d'un produit $PCj$ sera identifié comme aberrant. si en éliminant ce point. la variation du volume ou de la surface de la région $Rj$ est supérieure à un certain pourcentage (par exemple 10%). Il est préférable que les points aberrants détectés soient éliminés de la base de données. Leur élimination permet de filtrer la base. La méthode statistique utilisée est ensuite réappliquée sur la base filtrée.

## REGLES D'AFFECTATION

Il ne suffit pas de déterminer des régions et/ou des sous-espaces correspondant aux différentes classes, il est aussi nécessaire d'établir une règle permettant d'affecter un échantillon inconnu à une de ces classes car *a priori* les données représentant cet échantillon inconnu pourraient très bien correspondre à un point se trouvant entre deux classes. Au lieu de choisir une règle d'affectation fixe, la présente invention prévoit l'existence d'une pluralité de règles différentes, le choix de règle appropriée étant effectué au cours de la phase d'apprentissage de l'appareil. Les règles d'affectation préférées selon l'invention seront maintenant décrites.

A un échantillon inconnu $i$, on associe un point $x(i,j)$ du sous espace contenant la région spécifique du produit

$PC_j$. Cette région étant décrite dans un sous espace $SE_j$ particulier, *x(i,j)* est la projection du vecteur des mesures enregistrées sur ce sous espace. Selon la présente invention, il y a trois règles préférées pour affecter ce point à une classe particulière reconnue par l'appareil. Ces trois règles d'affectation consistent, respectivement, en une règle basée sur un critère géométrique (1), une règle basée sur un critère flou lié à la frontière délimitant chaque classe (2), et une règle basée sur un critère probabilistique (3).

**1. Critère géométrique (critère (1)) : Distance au centre**

L'ensemble des points représentant les échantillons extraits d'un même produit sont représentés dans la région associée à ce dernier. Le centre de gravité, $g_j$, de ses points représente le produit. On note $g_j$ le représentant du produit $PC_j$ dans la région associée à ce dernier.

$$g_j = \sum_l \frac{xl}{mj}$$

*xl* est le vecteur représentant un échantillon *l* du produit $PC_j$ et *mj* est le nombre des échantillons de ce produit.
Par, suite, un échantillon inconnu est identifié au produit dont il est le plus proche. C'est-à-dire qu'un échantillon inconnu i est identifié au produit $PC_k$ si

$$dis(x(i,k), g_k) = \min (dis(x(i,j), g_j)/j = 1;n)$$

*dis(x(i,j),g_j)* est la distance entre *x(i,j)* et $g_j$.

**2. Critère flou (critère (2)) : Frontière de la région**

Chaque région $R_j$ spécifique à un produit est délimitée par une frontière. Cette frontière est décrite par l'enveloppe convexe des points représentant les échantillons d'apprentissage. L'un des algorithmes proposés dans la littérature est celui donné par B. Chazelle dans ("Discrète Computational Geometry". 10, pp. 377-409 (1993)). Il permet de calculer de telles enveloppes dans un espace multidimensionnel.
Sur la figure 3 sont représentées les frontières des différentes régions représentées sur la figure 2.
Un échantillon inconnu *i* sera identifié à un produit avec un certain degré, selon sa position relative à cette frontière. Plus particulièrement, l'échantillon i sera affecté à une classe k si le degré d'expansion du volume/de la frontière de la région $R_k$ nécessaire pour englober le point représentant l'échantillon *i* est le minimum pour toutes les régions $R_j$. On note $V_j$ le volume ou la surface de la région délimitée par cette frontière et $\Delta$*(x(i,j),Vj)* sa variation en considérant *x(i,j)* comme élément de $PC_j$. Le degré d'appartenance est défini par :

$$Deg(x(i,j),Vj) = 100 \left\{1 - \frac{(\Delta x(i,j), Vj)}{Vj + \Delta(x(i,j),Vj)}\right\}$$

Un échantillon inconnu est alors identifié au produit $PC_k$ si

$$Deg(x(i,k),Vk) = \max(Deg(x(i,j),Vj)/j = 1;n)$$

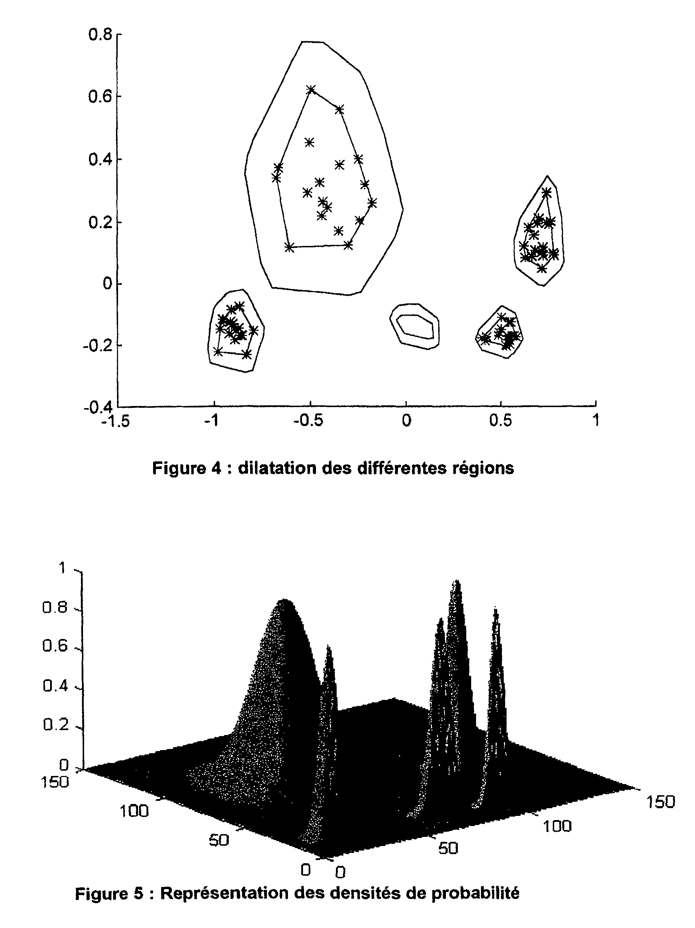

Par définition de l'enveloppe convexe, on déduit que si X(i,k) est à l'intérieur de la frontière alors la variation $\Delta$(x(i,j),Vj) est nulle. Par conséquent, le degré d'appartenance varie entre 0 et 100.
Afin de tenir compte des faibles variations de mesure, chaque région peut être dilatée tel qu'indiqué sur la Figure 4. Le coefficient de dilatation est fonction du volume ou de la surface de cette surface.

**3. Critère Probabiliste (critère (3)) : Densité de probabilité**

A chaque région est associée une fonction de densité de probabilité d'une loi normale Fj. Cette fonction est obtenue en recherchant la fonction F densité de probabilité du nuage de points. Le nuage étant hétérogène, cette fonction est modélisée en une somme pondérée de n gaussiennes. On a donc :

$$F(x) = \Sigma \alpha jFj(x) \text{ avec } j = \{1;n\} \text{ et } \Sigma \alpha j = 1$$

Fj étant une gaussienne, elle est définie par un vecteur de moyenne uj et une matrice de covariances Wj. Son expression est donnée par la formule:

$$Fj(x) = \frac{\exp^{(-(x-uj)'Wj^{-1}(x-uj)/2)}}{\sqrt[p]{2\pi}\ \sqrt{\det(Wj)}}$$

Les différents paramètres ($\alpha j$, uj, Wj) sont estimés par un algorithme de type EM (Estimation maximisation) (voir R.A. Redner et H.F. Walker, SIAM review, 26, pp. 195-239 (1984)).

Un échantillon inconnu est alors identifié au produit $PC_k$ si

$$Fk(x(i,k)) = \max (\alpha jFj(x(i,j))/j = 1;n)$$

Sur la figure 5, on a représenté les valeurs prises par les différentes densités sur les régions *Rj* de la figure 3, ainsi que sur leurs alentours. En normalisant les différentes fonctions de densités de probabilités, cette troisième règle d'affectation peut être considérée comme une règle d'affectation floue.

**Adaptation des critères**

Le critère d'affectation est choisi en fonction des applications et des formes des régions associées aux différents produits.

1. Le critère de distance est bien adapté aux structures de formes sphériques. Ceci est le cas lorsque les variations des mesures enregistrées par les différents capteurs sont de même ordre.
2. Le critère flou peut être utilisé dans n'importe quel cas de figure lorsque le volume de la région associée à chaque produit est différent de zéro. La dilatation de la frontière permet de tenir compte des faibles variations sur les mesures.
3. Comme la fonction de densité de probabilité doit être approchée, le nombre d'échantillons de chaque produit analysé doit être important. La normalisation de chacune de ces fonctions, c'est-à-dire, la transformation de chacune de ces fonctions en une fonction variant de 0 à 1 permet de définir une règle d'affectation floue.

**Choix du modèle**

Un modèle d'identification est constitué d'une méthode d'extraction de l'information (notamment l'ACP ou l'AFD) et d'un critère d'identification. On le note Modèle (méthode. critère). Selon les applications et les formes des structures des différentes régions, un critère est plus ou moins adapté qu'un autre. Une procédure de choix est donc nécessaire.

La procédure préférée pour réaliser ce choix consiste, dans un premier temps, à partager la base d'apprentissage en deux bases Bc et Bi, la première base servira à la recherche des régions spécifiques correspondant aux classes différentes et la deuxième base au choix du critère. Comme on connaît les produits dont sont extraits tous les échantillons des deux bases, le critère qui sera choisi est celui qui permet d'identifier le maximum des échantillons de Bi.

La repartition d'échantillons entre les bases Bi et Bc ne suit pas de règle particulière. Cependant, des représentations graphiques des points de mesure peuvent être affichées pour permettre à l'utilisateur de sélectionner pour la base Bc les points les plus proches les uns des autres (c'est-à-dire, des points bien regroupés dans les différentes classes). Compte tenu du fait que les bases doivent être partagées et que le modèle d'identification approprié est choisi sur la base des analyses qui sont réalisées sur les bases Bi et Bc, il est nécessaire d'avoir, dans chaque base, des données relatives à des classes dont les nombres d'échantillons sont d'une part significatif et d'autre part équilibrés.

En notant méthode (1) l'ACP et méthode (2) l'AFD, le modèle (modèle (méthode( 1 ), critère (k))) choisi est tel que

$$P(\text{méthode}( I ),\text{critère}(k)) = \max(P(\text{méthode }(1),\text{critère }(j))),$$

avec *i* = 1;2 et *j* = 1;3.

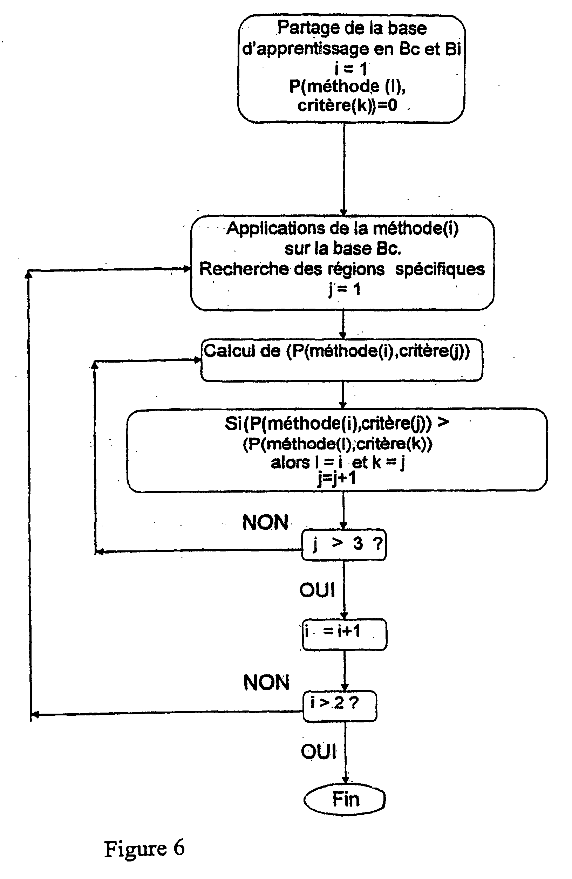

P(méthode(*i*),critère(*j*))) est le pourcentage des échantillons de la base *Bi* bien classés en utilisant le critère *j* sur les régions définies par la méthode *i*. L'organigramme de la Figure 6 résume cette procédure.

Bien que des modes de réalisation préférés de la présente invention aient été présentés ci-dessus, il convient de rappeler que la présente invention n'est pas limitée à ces modes de réalisation, décrits en tant qu'exemples non limitatifs.

Par exemple, le modèle d'identification peut être modifié pendant la phase d'identification. Les signaux de sortie des capteurs relatifs à un échantillon identifié en tant que membre d'une classe particulière avec un degré de certainté suffisamment significatif peuvent être injectés dans la base de données établie au cours de la phase d'apprentissage. L'analyse est alors refaite en tenant compte de cet échantillon. Ce modèle peut être très intéressant puisqu'il est évolutif et tient compte des faibles variations de mesures dues à des paramètres difficilement contrôlables ou au vieillissement des capteurs.

## Revendications

1. Appareil de classification, notamment destiné à une utilisation dans la reconnaissance ou la caractérisation d'odeurs, comprenant :

   un moyen destiné à acquérir, par une pluralité de canaux au cours d'une phase d'apprentissage, des données brutes représentant une pluralité d'instances d'une pluralité de classes différentes, l'affectation aux classes respectives des instances analysées au cours de la phase d'apprentissage étant connue ;
   une unité de traitement destinée à traiter les données fournies par le moyen d'acquisition de données pour déterminer, au cours de la phase d'apprentissage, un modèle d'identification comportant des définitions des classes différentes, et, pendant une phase d'identification, à classer selon une règle particulière une instance de classe inconnue dans une des classes définies au cours de la phase d'apprentissage ;

   **caractérisé en ce que** l'unité de traitement est adaptée à décider lequel d'une pluralité de différents modèles d'identification possibles, dont les caractéristiques sont stockées dans un répertoire, doit être appliqué.

2. Appareil de classification selon la revendication 1, **caractérisé en ce que** l'unité de traitement sélectionne une règle d'affectation en choisissant dans un répertoire une règle parmi une pluralité de différentes règles possibles, la règle d'affectation sélectionnée faisant partie du modèle d'identification déterminé par l'unité de traitement au cours de la phase d'apprentissage.

3. Appareil de classification selon la revendication 1 ou 2, **caractérisé en ce que** l'unité de traitement sélectionne une méthode d'extraction d'informations des données brutes en choisissant dans un répertoire une méthode parmi une pluralité de différentes méthodes possibles, la méthode d'extraction sélectionnée faisant partie du modèle d'identification déterminé par l'unité de traitement au cours de la phase d'apprentissage.

4. Appareil de classification selon la revendication 1, 2 ou 3, **caractérisé en ce que** l'unité de traitement établit plusieurs modèles d'identification candidats sur la base de données concernant un pourcentage seulement des instances traitées au cours de la phase d'apprentissage et sélectionne parmi ces modèles d'identification candidats le modèle qui donne le meilleur pourcentage d'affectations correctes quand des données représentant d'autres instances traitées au cours de la phase d'apprentissage lui sont soumises.

5. Appareil de classification selon l'une quelconque des revendications 1 à 4, **caractérisé en ce que** l'unité de traitement calcule pour chaque instance un vecteur *x* le représentant, et l'une des règles d'affectation applicable par l'unité de traitement consiste en l'identification de la classe *k* à laquelle se rapproche le plus le vecteur $x_i$ représentant une instance *i* de classe inconnue selon la formule suivante :

$$dis(x(i,k), g_k) = \min (dis(x(i,j), g_j)/j = 1;n)$$

où *x(i,j)* est la projection du vecteur $x_i$ sur un sous espace *SEj* dans lequel la classe j est bien discriminée par rapport aux autres classes, *dis(x(i,j),g_j)* est la distance entre *x(i,j)* et $g_j$, et $g_j$ est un point représentant la classe *j* et calculé selon la formule:

$$g_j = \sum_1 \frac{xl}{mj}$$

où *l* est une instance de la classe *j*, et *mj* est le nombre des instances de cette classe utilisées pour établir la définition de la classe.

6. Appareil de classification selon l'une quelconque des revendications 1 à 5, **caractérisé en ce que** l'unité de traitement calcule pour chaque instance un vecteur *x* le représentant, et l'une des règles d'affectation du répertoire consiste en l'identification pour chaque classe (*j*) du volume ou de la surface (*Vj*) de la frontière de la région (*Rj*) regroupant les instances de cette classe dans un sous-espace (*SEj*) dans lequel cette classe est bien discriminée par rapport aux autres, et à affecter une instance *i* de classe inconnue à une classe particulière (*k*) lorsque:

$$Deg(x(i,k),Vk) = max(Deg(x(i,j),Vj)/j = 1;n)$$

où Deg (x(i,j),Vj) est le degré d'appartenance de l'instance *i* à la classe *j* calculé selon la formule suivante:

$$Deg(x(i,j),Vj) = 100 \times \{1 - \frac{\Delta(x(i,j),Vj)}{Vj + \Delta(x(i,j),Vj)}\}$$

et $\Delta$(x(i,j),Vj) est la variation du volume ou de la surface de la région *Rj* lorsque la projection (*x(i,j)*) du vecteur (*x_i*) représentant l'instance (*i*) de classe inconnue est considérée comme étant un membre de la classe (*j*).

7. Appareil de classification selon l'une quelconque des revendications 1 à 6, **caractérisé en ce que** l'unité de traitement calcule pour chaque instance un vecteur *x* le représentant, et une des règles d'affectation du répertoire consiste en l'affectation d'une instance (i) de classe inconnue à une classe particulière (*k*) lorsque

$$Fk(x(i,k)) = max (Fj(x(i,j))/j = 1;n)$$

où Fj(x(i,j)) est une fonction de densité de probabilités calculée pour la classe *j* selon la formule suivante :

$$Fj(x) = \frac{\exp^{(-(x-uj)'Wj^{-1}(x-uj)/2)}}{\sqrt[p]{2\pi}\sqrt{\det(Wj)}}$$

8. Appareil de classification selon l'une quelconque des revendications 3 à 7, **caractérisé en ce que** l'une des méthodes d'extraction d'informations consiste en une analyse en composantes principales.

9. Appareil de classification selon l'une quelconque des revendications 3 à 7, **caractérisé en ce que** l'une des méthodes d'extraction d'informations consiste en une analyse factorielle discriminante.

10. Appareil de classification selon l'une quelconque des revendications précédentes. **caractérisé en ce que** l'unité de traitement des données brutes est adaptée à identifier les canaux dont les données n'aident pas à la différenciation des instances de classes différentes, et à déterminer le modèle d'identification à partir de données qui excluent les données provenant du canal ou des canaux ainsi identifiés.

11. Appareil de classification selon l'une quelconque des revendications précédentes. **caractérisé en ce que** l'unité de traitement des données brutes est adaptée à identifier des instances aberrantes d'une classe, pour lesquelles les coordonnées définissent des points qui s'éloignent des points correspondants aux autres instances de la classe d'une valeur dépassant une valeur de seuil, et à déterminer le modèle d'identification à partir de données qui excluent les données relatives aux instances aberrantes ainsi identifiées.

**Claims**

1.  Classification apparatus, notably for use in the recognition or the characterisation of odours, comprising:

    means for acquiring, by a plurality of channels during a learning phase, raw data representing a plurality of instances of a plurality of different classes, it being known to which classes the instances analysed during the learning phase belong;
    a processing unit for processing the data provided by the data acquisition means so as to determine, during the learning phase, an identification model comprising definitions of the different classes and, during an identification phase, to classify, according to a particular rule, an instance of unknown class into one of the classes defined during the learning phase;

    **characterised in that** the processing unit is adapted to decide which of a plurality of different possible identification models, the characteristics of which are stored in a directory, should be applied.

2.  Classification apparatus according to claim 1, **characterised in that** the processing unit selects an allocation rule by choosing in a list one rule from among a plurality of different possible rules, the selected allocation rule forming part of the identification model determined by the processing unit during the learning phase.

3.  Classification apparatus according to claim 1 or 2, **characterised in that** the processing unit selects a method of extracting information from the raw data by choosing from a list one method from among a plurality of different possible methods, the selected extraction method forming part of the identification model determined by the processing unit during the learning phase.

4.  Classification apparatus according to claim 1, 2 or 3, **characterised in that** the processing unit establishes several candidate identification models based on data concerning only a percentage of the instances processed during the learning phase and selects from amongst these candidate identification models the model which gives the best percentage of correct classifications when data representing other instances processed during the learning phase is submitted thereto.

5.  Classification apparatus according to any one of claims 1 to 4, **characterised in that** the processing unit calculates for each instance a vector $x$ representing it, and one of the allocation rules applicable by the processing unit consists in the identification of the class $k$ to which the vector $x_i$ representing an instance of unknown class is closest according to the following formula:

$$dis(x(i,k),g_k) = min \ (dis(x(i,j),gj)/j = 1;n)$$

    where $(x(i,j)$ is the projection of the vector $x_i$ onto a sub-space $SEj$ in which the class j is well-discriminated with respect to the other classes, $dis(x(i,j),g_j$ is the distance between $x(i,j)$ and $g_j$, and $g_j$ is a point representing the class $j$ and calculated according to the formula:

$$g_j = \quad \sum_{l} \ \textbf{Erreur !}$$

    where $l$ is an instance of the class $j$, and $mj$ is the number of instances of this class used to establish the definition of the class.

6.  Classification apparatus according to any one of claims 1 to 5, **characterised in that** the processing unit calculates for each instance a vector $x$ representing it, and one of the allocation rules of the list consists in the identification for each class ($j$) of the volume or the surface area ($Vj$) of the boundary of the region ($Rj$)grouping together the instance of this class in a sub-space ($SEj$) in which this class is well-discriminated from the others, and in allocating an instance $i$ of unknown class to a particular class ($k$) when:

$$Deg(x(i,k), Vk) = max(Deg(x(i,j), Vj)/j=1;n)$$

where **Deg(x(i,j),Vj)** is the "degree of belonging" of the instance **i** to the class **j** calculated according to the following formula:

$$Deg(x(i,j),Vj) = 100 \times \{1 - \text{Erreur!}\}$$

and $\Delta$**x(i,j),Vj)** is the variation of the volume or of the surface area of the region **Rj** when the projection (**x(i,j)**) of the vector (**$x_i$**) representing the instance (**i**) of unknown class is considered as being a member of the class (j).

**7.** Classification apparatus according to any one of claims 1 to 6, **characterised in that** the processing unit calculates for each instance a vector **x** representing it, and one of the allocation rules of the list consists in the classification of an instance (**i**) of unknown class to a particular class (**k**) when:

$$Fk(x(i,k)) = max (Fj(x(i,j))/j=1;n)$$

where **Fj(x(i,j))** is a probability density function calculated for the class **j** based on the following formula:

$$Fj(x) = \text{Erreur!}$$

**8.** Classification apparatus according to any one of claims 3 to 7, **characterised in that** one of the information extraction methods consists in a principal component analysis.

**9.** Classification apparatus according to any one of claims 3 to 7, **characterised in that** one of the information extraction methods consists in a discriminant factorial analysis.

**10.** Classification apparatus according to any one of the preceding claims, **characterised in that** the unit processing raw data is adapted to identify channels the data of which does not help in differentiating the instances of different classes, and in determining the identification model based on data excluding the data from the channel or channels thus identified.

**11.** Classification apparatus according to any one of the preceding claims, **characterised in that** the unit processing raw data is adapted to identify abnormal instances of a class, for which the co-ordinates define points which are distant from the points corresponding to the other instances of the class by an amount exceeding a threshold value, and to determine the identification model based on data excluding the data relating to the thus-identified abnormal instances.

**Patentansprüche**

**1.** Klassifiziervorrichtung, insbesondere für eine Verwendung beim Erkennen oder bei der Charakterisierung von Gerüchen, umfassend:

eine Einrichtung, die dazu ausgebildet ist, über eine Mehrzahl von Kanälen im Zuge einer Lernphase Rohdaten zu erfassen, die eine Mehrzahl von Objekten aus einer Mehrzahl unterschiedlicher Klassen repräsentieren, wobei die Zuordnung der analysierten Objekte im Zuge der Lernphase zu den einzelnen Klassen bekannt ist; eine Behandlungseinheit, die dazu ausgebildet ist, die von der Datenerfassungseinrichtung gelieferten Daten zu behandeln, um im Zuge der Lernphase ein Identifikationsmodell festzulegen, welches Definitionen der verschiedenen Klassen enthält, und um im Zuge einer Identifikationsphase ein Objekt unbekannter Klasse gemäß einer speziellen Regel in eine der Klassen zu klassifizieren, die im Zuge der Lernphase definiert wurden;

**dadurch gekennzeichnet, daß** die Behandlungseinheit dazu ausgebildet ist, zu entscheiden, welches von einer Mehrzahl unterschiedlicher möglicher Identifikationsmodelle, deren Charakteristika in einem Verzeichnis abgespeichert sind, angewendet werden muß.

2. Vorrichtung nach Anspruch 1, **dadurch gekennzeichnet, dass** die Behandlungseinheit eine Zuordnungsregel auswählt, indem sie in dem Verzeichnis eine Regel aus einer Mehrzahl verschiedener möglicher Regeln auswählt, wobei die ausgewählte Zuordnungsregel einen Teil des Identifikationsmodells bildet, welches von der Behandlungseinheit im Zuge der Lernphase bestimmt wurde.

3. Vorrichtung nach Anspruch 1 oder 2, **dadurch gekennzeichnet, dass** die Behandlungseinheit ein Extraktionsverfahren zum Extrahieren von Informationen aus Rohdaten auswählt, indem sie in dem Verzeichnis ein Verfahren unter mehreren verschiedenen möglichen Verfahren auswählt, wobei das ausgewählte Extraktionsverfahren Teil des Identifikationsmodells ist, das von der Behandlungseinheit im Zuge der Lernphase bestimmt wurde.

4. Vorrichtung nach Anspruch 1, 2 oder 3, **dadurch gekennzeichnet, dass** die Behandlungseinheit mehrere Identifikationsmodell-Kandidaten auf der Grundlage von Daten aufstellt, die einen Prozentsatz von ausschließlich Objekten betreffen, die im Zuge der Lernphase behandelt wurden, und unter den Identifikationsmodell-Kandidaten dasjenige Modell auswählt, welches den größten Prozentsatz korrekter Zuordnungen ergibt, wenn ihm Daten zugrunde gelegt werden, die andere im Zuge der Lernphase behandelte Objekte darstellen.

5. Vorrichtung nach einem der Ansprüche 1 bis 4, **dadurch gekennzeichnet, dass** die Behandlungseinheit für jedes Objekt einen das Objekt repräsentierenden Vektor *x* berechnet und eine der Zuordnungsregeln, die von der Behandlungseinheit anwendbar ist, aus der Identifikation der Klasse *k* besteht, der sich der ein Objekt *i* einer unbekannten Klasse repräsentierende Vektor $x_i$ am stärksten nähert, entsprechend folgender Formel:

$$\text{dis}(x(i,k), g_k) = \min\,(\text{dis}(x(i,j), g_j/j = 1;n)$$

wobei *x(i,j)* die Projektion des Vektors $x_i$ auf einen Unterraum *SEj* ist, in welchem sich die Klasse j bezüglich anderer Klassen unterscheidet, *dis(x(i,j), $g_j$)* der Abstand zwischen *x(i,j)* und *$g_j$* ist, und *$g_j$* ein Punkt ist, der die Klasse *j* repräsentiert und gemäß folgender Formel berechnet wird:

$$g_j = \sum_l \frac{xl}{mj}$$

wobei *l* ein Objekt der Klasse *j*, und *mj* die Anzahl der Objekte dieser Klasse ist, die verwendet werden, um die Definition der Klasse zu bilden.

6. Vorrichtung nach einem der Ansprüche, **dadurch gekennzeichnet, dass** die Behandlungseinheit für jedes Objekt einen das Objekt repräsentierenden Vektor *x* berechnet, und eine der Zuordnungsregeln des Verzeichnisses besteht in der Identifikation für jede Klasse (*j*) des Volumens oder der Oberfläche (*Vj*) der Grenze der Zone (*Rj*), welche die Objekte dieser Klasse in einem Unterraum (*SEj*) neu gruppiert, in welchem diese Klasse gegenüber anderen Klassen deutlich unterschieden ist, und in dem Zuordnen eines Objekts *i* unbekannter Klasse zu einer speziellen Klasse (*k*), wenn:

$$\text{Deg}(x(i,k), Vk) = \max(\text{Deg}(x(i,j), Vj)/j = 1;n)$$

wobei Deg(x(i,j), Vj) das Zugehörigkeitsmaß des Objekts *i* zu der Klasse *j* ist, berechnet gemäß folgender Formel:

$$\text{Deg}(x(i,j),Vj) = 100 \times \left\{1 - \frac{\Delta(x(i,j),Vj)}{Vj + \Delta(x(i,j),Vj)}\right\}$$

und $\Delta(x(i,j), Vj)$ die Variation des Volumens oder der Oberfläche der Zone *Rj* ist, wenn die Projektion (x(i,j)) des das Objekt (*i*) unbekannter Klasse repräsentierenden Vektors (*$x_i$*) als ein Element der Klasse (*j*) betrachtet wird.

**7.** Vorrichtung nach einem der Ansprüche 1 bis 6, **dadurch gekennzeichnet, dass** die Behandlungseinheit für jedes Objekt einen das Objekt repräsentierenden Vektor *x* berechnet, und eine der Zuordnungsregeln des Verzeichnisses besteht in der Zuordnung eines Objekts (*i*) unbekannter Klasse zu einer speziellen Klasse (*k*), wenn

$$Fk(x(i,k)) = \max (Fj(x(i,j))/j = 1;n)$$

wobei Fj(x(i,j)) eine Funktion der Wahrscheinlichkeitsdichte ist, die für die Klasse *j* gemäß folgender Formel berechnet wird:

$$Fj(x) = \frac{\exp^{(-(x-uj)'Wj^{-1}(x-uj)/2)}}{\sqrt[\ell]{2\pi}\sqrt{\det(Wj)}}$$

**8.** Vorrichtung nach einem der Ansprüche 3 bis 7, **dadurch gekennzeichnet, dass** eines der Informations-Extraktionsverfahren in einer Analyse der Hauptkomponenten besteht.

**9.** Vorrichtung nach einem der Ansprüche 3 bis 7, **dadurch gekennzeichnet, dass** eines der Informations-Extraktionsverfahren in einer faktoriellen diskreminanten Analyse besteht.

**10.** Vorrichtung nach einem der vorhergehenden Ansprüche, **dadurch gekennzeichnet, dass** die Behandlungseinheit für die Rohdaten dazu ausgebildet ist, diejenigen Kanäle zu identifizieren, deren Daten keinen Beitrag leisten zu der Unterscheidung der Objekte der verschiedenen Klassen, und das Identifikationsmodell ausgehend von Daten zu bestimmen, die solche Daten ausschließen, die aus einem solchen Kanal oder solchen Kanälen stammen.

**11.** Vorrichtung nach einem der vorausgehenden Ansprüche, **dadurch gekennzeichnet, dass** die Behandlungseinheit für Rohdaten dazu ausgebildet ist, Ausreißer-Objekte einer Klasse zu identifizieren, für die die Koordinaten Punkte definieren, die von anderen Objekte der Klasse entsprechenden Punkten um einen Wert beabstandet sind, die über einem Schwellenwert liegen, und das Identifikationsmodell anhand von Daten zu bestimmen, die Daten ausschließen, die sich auf derart identifizierte Ausreißer-Objekte beziehen.

EP 1 007 961 B1

L'Etape 1
Acquisition des Données
sur des échantillons de n produits

L'Etape 2
Extraction du maximum d'information
Application de l'ACP ou l' AFD .
Initilisation j = 1

L'Etape 3
Recherche de la région Rj
qui discrimine au mieux le produit PCj

L'Etape 4
j = j+1    NON    j = n ?

OUI

Fin

**Figure 1 : Recherche des régions spécifiques**

**Figure 2 : Plan Discriminant**

**Figure 3 : enveloppes convexes délimitant les différentes régions**

**Figure 4 : dilatation des différentes régions**

**Figure 5 : Représentation des densités de probabilité**

Partage de la base
d'apprentissage en Bc et Bi
i = 1
P(méthode (l),
critère(k))=0

Applications de la méthode(i)
sur la base Bc.
Recherche des régions spécifiques
j = 1

Calcul de (P(méthode(i),critère(j))

Si(P(méthode(i),critère(j)) >
(P(méthode(l),critère(k))
alors l = i et k = j
j=j+1

NON

j > 3 ?

OUI

i = i+1

NON

i > 2 ?

OUI

Fin

Figure 6